# EUROPEAN PATENT APPLICATION

(11) **EP 1 595 527 A2**
(43) Date of publication of application: **16.11.2005**
(21) Application number: 05015361.8
(22) Date of filing: 02.03.2001
(51) Int. Cl.: A61K 7/42

(54) **Micronized zinc oxide skin protector formulation**

(30) Priority: 03.03.2000 US 186624 P
(62) Divisional of application: 01923320.4
(71) Applicant: Australian Importers, Ltd., Las Vegas, NV 89129 (US)
(72) Inventor: Vromen, Jacob, Botany New South Wales 2019 (AU)
(74) Representative: Schnappauf, Georg

(57) **Abstract**

Micronized zinc oxide skin protector formulations comprising between about 8 % and 40 % zinc oxide. These compositions preferably comprise micronized zinc oxide, preferably obtained via a "wet" micronization process in oil, and also preferably include agents that block or screen light in the visible and/or infrared ranges, and also preferably include hydrotropes and humectants to aid in the dissipation of heat caused by the irradiation of light in the visible and/or infrared ranges, and alleviate the photo-damage and/or heat damage associated with such irradiation. These compositions are preferably readily absorbable, transparent, non-irritating, non-comedogenic and are hypo-allergenic. The compositions may comprise additional ingredients such as biological additives (e.g., botanicals and herbals).

## Description

### Background of the Invention

### Field of the Invention

The invention provides skin protector formulations, often referred to as sunscreen formulations. More particularly, the invention provides skin protector formulations which comprise between about 8% and about 30% by weight micronized metal oxide, preferably zinc oxide (ZnO), in a formulation that is cosmetically acceptable for application to the face, or other exposed areas of the skin. Even more particularly, the invention provides skin protectors for preventing against and for treating the aging or photodamage of skin through exposure to sunlight or other sources of light in the ultraviolet (UV), visible, and infrared (IR) ranges.

### Description of the Related Art

There are two predominant types of skin protecting agents, chemical absorbers and physical barriers, each of which offers certain advantages, and presents certain limitations and disadvantages. Chemical absorbers (also known as chemical filters) are compounds that are absorbed through the skin and exert their beneficial effects through a chemical process of absorbing certain wavelengths of light, most typically UV irradiation, i.e. light having wavelengths equal to or shorter than approximately 400 nm, and more specifically, having wavelengths of approximately 100 nm to approximately 400nm. These compounds (e.g., paraamino benzoic acid (PABA) and its derivatives, oxybenzone, octyl methoxycinnamates, benzophenones) have been used for many years as sole chemical absorbers, primarily because they do not create a whitening effect. The lack of such a whitening effect makes them cosmetically acceptable.

Although these compounds offer a measure of protection against ultraviolet-B (UV-B) irradiation, they provide incomplete protection against the entire ultraviolet-A (UV-A) range. In addition, some chemical filters degrade after prolonged sun exposure, through a process known as photolysis, and therefore tend to lose their efficacy over time. Further, chemical absorbers such as PABA can irritate sensitive skin. Most chemical absorbers have the advantage of fast absorption, superior UV-B protection and cosmetic elegance in a broad spectrum, cosmetically acceptable formula.

Physical barriers provide either a visible or invisible protective barrier to the skin, preventing the penetration of UV irradiation. Two effective mineral filters used as physical barriers are zinc oxide and titanium dioxide. Among the known chemical absorbers and physical barrier compounds, only microfine zinc oxide has been clinically shown to protect the skin from the entire UV spectrum (including the entire UV-A and UV-B ranges), even when used during prolonged sun exposure, including the highest and broadest protection against UV-A rays, which are believed to be responsible for the effects of photo-aging.

Most early commercially available zinc oxide sunscreen formulations consisted of large particles, and were therefore difficult to apply, and had a dramatic whitening effect and were therefore comedogenic, meaning that they tend to clog skin pores. Later zinc oxide sunscreen formulations include micronized zinc oxide (*i*.*e*., zinc oxide milled into fine particles having mean particle sizes on the order of microns) made via conventional micronization processes. These formulation provided physical barriers that were cosmetically acceptable and were non-comedogenic. However, the maximum amount of zinc oxide in currently commercially available formulations is no more than about six to about seven percent, by weight, and these compositions must be vigorously rubbed onto the skin to obtain transparence and a suitable cosmetic effect.

It is not currently appreciated by those of skill in the art that exposure to light in the visible range (i.e., light having wavelengths of between approximately 400 nm and approximately 700 nm) and/or exposure to light in the infrared (IR) range (i.e, light having wavelengths equal to or greater than approximately 700 nm, and more specifically, having wavelengths of approximately 700 nm to approximately 1000 nm) are more harmful than exposure to UV irradiation, and may be a more direct cause of skin aging, the photodamage of skin, heat damage to skin, and other skin surface maladies, including skin cancer.

There is accordingly a need a for sunscreen formulation that contains higher amounts of zinc oxide, and that retains its transparence upon application to the skin, and that does not require vigorous rubbing onto the skin to obtain transparence and cosmetic acceptability. There is also a need for formulations that include agents that block, and provide a barrier against, penetration of light in the IR and visible ranges, as well as the UV range, and provide protection against heat damage to skin. The invention addresses one or more of these needs.

### Summary of the Invention

A topical sunscreen composition comprising between about 8% and about 40% by weight micronized zinc oxide is disclosed. Preferably, this composition further comprises a hydrotrope and water. The zinc oxide is preferably micronized by a "wet" micronization process and the mean particle size of the zinc oxide is less than approximately 5 µm, or is between approximately 0.01 µm and approximately 1 µm. Also preferably, the composition further comprises a biological additive or combination thereof, preferably including the biological additive oil of Melaleuca alternifolia (Tea Tree Oil), and preferably further comprising iron (III) oxide, titanium oxide, or a combination thereof. The composition preferably comprises about 10%, 15%, 20%, 25%, 30%, 35%, or 40% or more, by weight, micronized zinc oxide. The wet micronization for preparing the zinc oxide is preferably performed in an oil, which is preferably derived from plant materials. This oil preferably comprises capric/caprylic triglycerides. The compositions described herein provide cosmetically acceptable means for protecting skin against the harmful effects of UV, visible, and IR irradiation without the need for chemical absorbers. A method of providing UV protection to a mammal in need thereof, comprising the step of topically applying the composition to the mammal, including, preferably domesticated animals or humans, is also disclosed. A method for treating an individual having prematurely aged, photo-damaged or heat damaged skin comprising: diagnosing a individual having said skin condition, and applying to the skin the composition is also disclosed.

### Brief Description of the Drawings

Fig. 1 illustrates a skin protector designated "Summer Lotion" plus iron (III) oxide and illustrates a plot of light absorbance as a function of wavelength.
Fig. 2 illustrates the ability of two micronized zinc oxide formulations B17 lotion (comprising 10% ZnO, 2.5% TiO₂) (SN 2200) and B18 lotion (comprising 10% ZnO, 2.5% TiO₂, and 2% ZnO/Fe₂O₃) (SN2221) to block and/or absorb wavelengths in the IR, visible, and UV ranges.
Fig. 3 illustrates the ability of two micronized zinc oxide formulations designated "Zinc pink"-brand (comprising 32% ZnO, 1% Fe₂O₃) (SN 2222) and "Zince"-brand (comprising 32% ZnO, 1% Fe₂O₃, 4% TiO₂) (SN2223) to block and/or absorb wavelengths in the IR, visible, and UV ranges.
Fig. 4 illustrates the ability of two micronized zinc oxide formulations designated "Zinc pink"-brand (comprising 32% ZnO, 1% Fe₂O₃) (SN 2222) and "Zince"-brand (comprising 32% ZnO, 1% Fe₂O₃, 4% TiO₂) (SN2223) to block and/or absorb wavelengths in the IR, visible, and UV ranges.
Fig. 5 illustrates the ability of two micronized zinc oxide formulations designated "Zinc pink"-brand (comprising 32% ZnO, 1% Fe₂O₃) (SN 2222) and "Zince"-brand (comprising 32% ZnO, 1% Fe₂O₃, 4% TiO₂) (SN2223) to block and/or absorb wavelengths in the IR, visible, and UV ranges.
Fig. 6 illustratess the ability of a micronized zinc oxide formulation designated SC74-1 Gel Base (comprising 7% ZnO, 5% TiO_{2,}, 1% Fe₂O₃) (20 micron wet film) to block and/or absorb wavelengths in the IR, visible, and UV ranges.

### Detailed Description of the Preferred Embodiment

The invention is, in part, based on a recognition that UV irradiation is not the single, nor is it necessarily the most harmful, irradiation to skin in the context of causing premature skin aging, sun- or photo-damage to skin and other skin maladies such as skin cancer. Rather, skin is damaged by exposure to the visible and infrared (IR) ranges of the spectrum, and related photodamage, and heat damage to the skin caused by such exposure. Accordingly, to provide optimum protection from heat damage attributable to exposure to infrared light, composition, including lotions, are provided that comprise micronized zinc oxide, which blocks total UV plus light in the visible range, preferably in amounts higher than previously available in a cosmetically acceptable formulation. These compositions also preferably comprise titanium oxide (TiO₂) which blocks UV-B and iron (III) oxide (Fe₃O₂), also known as hematite, which block well into the IR range. To maintain cosmetic acceptability of the composition, relatively small amounts of iron (III) oxide are preferably included. Such amounts generally include amounts up to and including approximately 1 %, by weight.

Improved micronized topical zinc oxide skin protecting compositions for topical application are provided. These compositions comprise between about 8% and about 30% percent, by weight, and preferably between about 10% and about 20% by weight, and more preferably between about 10% and 15% by weight, micronized zinc oxide. In a particularly preferred embodiment, the composition comprises about 12.5% by weight micronized zinc oxide. These formulations are preferably water resistant; are preferably sweat-proof; are preferably non-irritating; are preferably hypo-allergenic; are preferably non-whitening; are preferably easily absorbed into the skin; are preferably transparent; are preferably cosmetically elegant; are preferably non-comedogenic (will not clog pores); and preferably provide maximum, broad spectrum photoprotection and provide a therapeutic or curative benefit for sun-damaged or photosensitive skin. Other metal oxides, such as titanium oxide and iron (III) oxide may also be included in the composition.

The compositions are designed so that they may be worn daily, may be worn under make-up, and are well tolerated on even the most sensitive and reactive skin. The compositions may be topically applied to a mammal, including horses, cat and dogs and other domestic animals, and most preferably to a human. Preferably, the compositions may be topically applied to any portion of the skin that will be or will tend to be exposed to UV irradiation, including, but not limited to, the face, the ears, the scalp, the hands, arms, shoulders, legs, feet, abdomen and back, and any area of the skin that an individual chooses to expose to UV, visible, and/or IR irradiation. Such UV irradiation is typically, but not necessarily, directed to the skin from the sun. Other UV, visible, and IR range light sources include most typical UV light sources, as will be appreciated by those of skill in the art, and also including most industrial light sources.

The compositions described herein provide cosmetically acceptable means for protecting skin against the harmful effects of UV, visible, and IR irradiation without the need for chemical absorbers. Additionally, the compositions described herein provide protection against skin drying and the associated damage through the use of hydrotropes, humectants, and/or emollients. Specifically, these compositions provide protection well into the IR range, including light having wavelengths ranging from approximately 190 nm to approximately 790 nm, and provide absorbance of light having wavelengths ranging from approximately 190 nm to approximately 390 nm, and reflectance of light having wavelengths ranging from approximately 190 nm to approximately 790 nm.

Preferably, the compositions and formulations are applied directly to the skin once per week, once per day or three times per day. Alternatively, these compositions and formulations may be applied directly to the skin less frequently or only on specific occasions, for example, before extended exposure to UV, visible, and IR range irradiation, to achieve certain of the benefits described herein. The quantity and extent of application will vary with the particular amount of time an individual is exposed to UV irradiation.

The zinc oxide, which may be obtained as a coarse powder form, can be micronized in the dry state as is conventional, or can be "wet" micronized in an oil, preferably a vegetable oil, and most preferably capric/caprylic triglycerides, which breaks the zinc oxide into ultrafine particles and, at the same time, coats the particles with the oil, which promotes maximum absorbance stability, and tends to prevent agglomeration and coalescing of the zinc oxide.

The zinc oxide powder may be converted into the desired particulate size state by conventional methods, *e*.*g*. by grinding the powder, in coarse particle form, in the presence of suitable grinding aids and using known grinding apparatus, *e*.*g*., a jet, ball, vibration or hammer mill, preferably a high speed stirring mill or impact mill, especially a rotating ball mill, vibrating mill, tube mill or rod mill.

According to the preferred method of manufacturing the preferred formulations of the invention, zinc oxide in powder form is subjected to a "wet" micronization process, as made available, and preferably made by, by Microniser Pty. Ltd. of Dandenong, Australia / Micronisers of Australia of Melbourne, Australia. This process, which may be contrasted to so-called "dry" or standard micronization processes preferably involves the grinding of the powder, suspended or otherwise in the presence in a non-aqueous liquid, preferably an oil (hereinafter, the "suspending oil"). The process is preferably conducted in an abrasion-resistant container in the presence of a grinding medium, and at sufficiently high rpm for a sufficiently long duration, using a suitable stirrer. The resulting suspension may separated from the grinding medium by suction filtration of the powder. This micronization process is capable of producing particles of zinc oxide having a mean particle size corresponding to, and including mean particle sizes as small as, the molecular size of zinc oxide.

Alternatively, the grinding may be conducted in the presence of 0.1 to 30%, and preferably 0.5 to 15% by weight, of a grinding aid such as an alkylated vinylpyrrolidone polymer, a vinylpyrrolidone-vinylacetate copolymer, an acylglutamate, an acrylate-tert.-octylpropenamide copolymer, a ditolylether sulphonic acid-formaldehyde condensate, a Carbomer, a commercial mixture of fatty acid esters comprising a nonionic precursor such as tristyrylphonol ethoxylate or, in particular, a phospholipid, as described in U.S. Patent 5,869,030, the complete description of which is hereby incorporated by reference herein.

The suspending oil is most preferably a vegetable oil, most preferably capric/caprylic triglycerides, that promotes (along with the physical micronization process, as described above) breaking the metal oxide into ultrafine particles and, at the same time, coats the particles with the oil, which promotes maximum absorbance and stability of the metal oxide acid in the formulation. The micronized metal oxide particles preferably used in the invention preferably exhibit a mean particle size of no greater than approximately 5 µm, more preferably no greater than approximately 2 µm, and most preferably between approximately 0.01 µm and 1 µm, but may have mean particle size as large as about 50-800 nm, about 50-500 nm, or about 50-100 nm. The metal oxide most preferably comprises zinc oxide, and may also preferably comprise titanium oxide and iron (III) oxide, and combinations thereof.

Oils most preferable and therefor most suitable for use in the invention include caprylic triglycerides, capric triglycerides, isostearic triglycerides, adipic triglycerides, propylene glycol myristyl acetate, lanolin oil, polybutene, isopropyl palmitate, isopropyl myristate, diethyl sebacate, diisopropyl adipate, hexadecyl stearate, cetyl oleate, oleyl alcohol, hexadecyl alcohol, wheatgerm oil, vegetable oils such as castor oil, corn oil, cottonseed oil, olive oil, palm oil, coconut oil, palm kernel oil, canola oil, safflower oil, jojoba oil, hydrogenated vegetable oils, mineral oil and silicone oils. In a preferred embodiment, the zinc oxide is micronized in the presence of capric/caprylic glycerides in which the zinc oxide is present in an amount from 70% to 80%, by weight.

The zinc oxide compositions may be formulated for topical application with pharmaceutically acceptable carriers using methods well known in the cosmetic and pharmaceutical arts, including gels, creams, ointments, emulsions, dispersions, salves, pastes, lotions and the like. These formulations may additionally comprise one or more emulsifying agents (e.g., stearic acid, cetyl phosphate, cetyl alcohol), humectants (*e*.*g*., glycerin, glycerol, sorbitol and other polyols), surfactants (*e*.*g*., ceteth-20, laneth-40), colorants such as staining dyes and pigments (*e*.*g*., calcium, barium and aluminum lakes, iron oxides, titanium dioxide and mica), antioxidants (*i*.*e*., tocopherols, retinoids, ascorbyl palmitate, thiodipropionic acid), viscosity-enhancing agents (*e*.*g*., cetearyl alcohol, polyethylene glycol), optional, additional vitamins, optional, additional minerals, emollients (*e*.*g*., paraffin liquid, polysorbate-60, lanolin, stearyl octanoate), skin conditioning agents (*e*.*g*., propylene glycol, sweet almond oil, octyldodecyl neopentanoate, urea, lactic acid, allantoin), biological additives (*e*.*g*., botanicals and herbals), UV absorbers and/or physical barriers (*e*.*g*., octyl methoxycinnamate, benzophenone-4, butyl methoxydibenzolylmethane, oxybenzone, titanium dioxide), germicides (*e*.*g*., antibiotics, Triclosan), preservatives (*e*.*g*., BHA, BHT, methylparabon, ethylparaben, propylparaben, butylparaben, octhilinone, sodium benzoate, octhilinone, phenonip) and fragrances (*e*.*g*., strawberry extract, manfigera indica, natural rose, banana extract), binders (e.g., polyacrylamide/laureth-7) and pH adjusters (e.g., triethanolamine, phosphate buffer). It will be appreciated by those of skill in the art that particular compounds may be properly classified in one, or two or more of the abova-listed classifications or compound types.

The compositions may also include one or more biological additives, such as botanicals or herbals. As used herein, the term "biological additive" indicates any compound obtained from a natural source, including plants, animals, bacteria and yeast, which has a medicinal or otherwise beneficial effect when topically applied to the skin. Examples of biological additives include oil of *Melaleuca alternifolia*, oil of *Lavandula angustifolia, Carica papaya* extract, *Echinacea angustifolia* extract, *Mimosa tenuiflora* extract, *Hydrocotyl (centella) asiatica* extract, gingko biloba extract, oil of *Melaleuca alternifolia* (tea tree oil), *Matricaria chamomila* (chamomile) extract, *Hypericum perforatum* extract, *Aloe barbedensis* extract, and the like. The biological sources for "biological additive" may also include, but are not limited to the following: Aloe Vera, Aloe Barbedensis; Arnica, Arnica Montana; Bladderwrack (seaweed), Fucus Vesciculosis; Birch, Betula Alba (Pendula); Chamomile, Matricaria Chamomila (Chamomila Reculita); Marsh Mallow, Althea Officinalis; Meadow Sweet, Spirea Ulmaria (Filipendula); Mint/Lemon Balm, Melissa Officinalis; Mimosa, Mimosa Tenuiflora; Myrrh Tincture, Commiphor Myrrha; Neem, Melia Azadirachta; Nettle (stinging), Urtica Dioica; Papaya, Carica Papaya; Propolis (bee glue), Propolis Cera; Raspberry, Rubis Idaeus; Red Poppy, Papaver Rhosas; Rose Hip (dog rose), Rosa Canima; Rosemary, Rosemarinus Officinalis; Sage, Salvia Officinalis; St. Johns Wort, Hypericum Perforatum; Strawberry, Fragaria Vesca; Thea Sinensis (green tea), Camelia Sinensis; Walnut, Juglans Regia; Witchhazel (dist/extr), Hamamelis Virginiana; Yarrow, Achillea Millefolium; Wild Yam, Dioscorea Villosa; Hawthorn, Crataegus Monogina/Oxyantha; Herma (black/rod), Lawsoma Ehemus; Hops, Humulus Lupulus; Horse Chestnut, Aesculus Hippocastanum; Horse Tail, Equisitum Arvense; Ivy, Hedera Helix; Linden/Lime Tree Blossoms, Tilia Argentea Cordata; Madder, Rubia Tinctorum; Marigold, Calendula Officinalis; Centella Asiatica, Centella Asiatica Urban (hydrocotyl Asiatica); Carrot (roots), Daucus Carota; Comfrey (Allantoine), Symphytum Officinale; Coneflower (Echinacea), Echinacea Angustifolia; Cucumber, Cucumis Sativus (Frucus Cucumis); Fenugreek, Trigonella Foenum Greacum; Gingko, Gingko Biloba; Ginseng, Panax Ginseng; Great Burdock, Radix Bardanea/Arctium Lappa; Tea Tree Oil, Oil of Melaleuca Alternifolia; Colts Foot, Tussilago Farfara; Clover, Trifolium Pratense; Speedwell, Veronica Officinalis.

Further biological additives, along with the biological or medicinal properties of the biological additives described herein and of other known biological additives are know to those of skill in the art. References, including encyclopedias and treatises, known to those of skill in the art, that described such biological additives, along with the biological or medicinal properties of the biological additives described herein, include: Guenther - The Essential Oils, Van Nostrand; Int. Cosmetic Ingredient Dictionary, Vol 1 & 2, C.T.F.A. 1995; Int. Cosmetic Ingredient Handbook, C.T.F.A. 1995; British Herbal Pharmacopoeia, British Herbal Medicine Assoc., 1983; Clinical Applications of Ayurvedic & Chinese Herbs, K. Bone, Phytotherapy Press, 1996; A Handbook of Chinese Healing Herbs, D. Reed, Shambala, Boston, 1995; Echinacea - Nature's Immune Enhancer, S. Foster, Healing Arts Press, Rochester, 1991; Encyclopedia of Herbs, D. Brown, RD Press, 1995; Encyclopedia of Medicinal Plants, A. Chevalier, Dorling Kingers Ley, 1996; L'Angelica - Herbal Extracts; Cosmetochem - Herbasol Extracts. These references are incorporated herein in their entirety.

As used herein, the terms "photo aging" and "photo damage" refer to conditions caused, at least in part, by a heat reaction in the skin. This process is not unlike the loss of color fastness in a textile. It can be effectively protected against, and prevented, through the use of a so-called "super" moisturizer, which retains water in the skin through the action of hydrotropes and humectants; one of the principle roles of the oxide in the formulations described herein is to reflect light and to prevent the heating of the skin.

In a preferred embodiment, the composition comprises one or more moisturizers, hydrotropes, humectents and/or emollients that hydrate the skin and prevent drying. Hydrotropes are typically small, deep penetrating molecules that tend to bind water molecules to each hydrotrope molecule. Preferred hydrotrope tend to bind up to twelve (12) water molecules to each hydrotrope molecule. Examples of hydrotropes include, most preferably, urea, and the various chemical derivatives of urea, triethanal lactate/sodium lactate, sorbitol, glycerin, glucose, ethylene, diethylene, triethylene, polyglycol, propyleneglycol, mannitol, glucesides, hyaluronic acid, and larger molecules including mucopolysaccharides, chitin liquid-polysaccharide glucosamine, proteins, and collagen. The hydrotropes serve to protect against infrared (IR) irradiation, which tends to heat the skin, and is also present in sunlight and light from other broad spectrum light sources. More specifically, the water molecules bound to the hydrotrope serve to dissipate the heat generated by the IR irradiation.

As used herein, the term "hydrotrope" refers to compounds exhibiting the property of hydrotropism, the property of certain compounds, minerals and/or organic compounds, to increase the solubility in water of substances, which are difficult to dissolve or even to dissolve in soluable bodies. A hydrotropic compound is, each water soluable compound, ionized or not, which enhances dissolving power in water. The hydrophilic groups of a compound, which dissolves in water, will enter in residence with the water molecule by forming bridges or dipole systems through solvation and hydration. In a particularly preferred embodiment, the hydrotrope is urea, ferrous oxide, or a mixture thereof. The hydrotrope is present in an amount from about 1% to 20% by weight, more preferably between about 5% and 15% by weight, and most preferably about 10% by weight.

In a preferred embodiment, one or more emollients is present in the formulation in a combined amount of from about 1% to 20% by weight, more preferably from about 5% to 15%, and most preferably about 10% by weight.

In another preferred embodiment, one or more emulsifying agents is present in the formulation in a combined amount of from about 5% to 25% by weight, more preferably from about 10% to 20%, and most preferably about 15% by weight.

The composition may also comprise, in addition to zinc oxide, one or more sunscreen agents, either a chemical absorber, physical barrier, or combination thereof. In a preferred embodiment, this additional agent(s) is present in a combined amount of from about 1% to 10% by weight, more preferably about 2% to 8% by weight, and most preferably about 3% to 5% by weight.

In a preferred embodiment, one or more biological additives is present in a combined amount of from about 1% to 10% by weight, more preferably from about 3% to 8% by weight, and most preferably from about 5% to 7% by weight.

In another preferred embodiment, the composition comprises the biological additive oil of Melaleuca alternifolia (Tea Tree Oil) which has deep penetrating healing, soothing and antiseptic properties. This oil has also shown the potential to treat surface skin cancers in informal studies using ranchers in the Australian outback as test subjects. Clinical studies are currently planned and/or underway in the United States to further evaluate this curative potential.

### EXAMPLES

The following examples are illustrative, but not limiting, of the topical micronized zinc oxide sunscreen formulations described herein.

### EXAMPLE 1

A preferred cream formulation contains:

| Zinc oxide cream formulation | | |
|---|---|---|
| COMPOUND | WEIGHT % | TYPE OF COMPOUND |
| Deionized water | 10 | solvent |
| zinc oxide | 12.5 | skin protectant |
| Octyldodecyl neopentanoate | 7.5 | emollient, skin conditioning agent |
| Urea | 10 | skin conditioning agent, hydrotrope |
| Triethanolamine | 5 | pH adjuster |
| stearic acid | 4 | emulsifying agent |
| cetyl phosphate | 2.5 | emulsifying agent |
| Sorbitol | 4 | emulsifying agent |
| Lanolin | 3 | moisturizer |
| Octylmethoxy cinnamate | 3 | UV absorber |
| Glyceryl stearate | 2.5 | emulsifying agent |
| cetyl alcohol | 2.5 | emulsifying agent |
| Caprylic/capric triglycerides | 2.5 | skin conditioning agent |
| Dimethicone | 2.5 | skin conditioning agent |
| Benzophenone-4 | 2.5 | UV absorber |
| lactic acid | 2.5 | skin conditioning agent |
| laneth-40 | 2 | surfactant |
| stearyl octanoate | 2.5 | emollient |
| oil of Melaleuca alternifolia | 2 | biological additive |
| oil of Lavandula angustifolia | 3 | essential oil, fragrance |
| Mimosa tenuiflora extr. | 2 | biological additive |
| Aloe barbedensis extr. | 2 | biological additive |
| Allantoin | 0.2 | skin conditioning agent |
| methyl/ethyl/propyl/butyl parabens/phenoxyethanol | 0.4 | preservative |

### EXAMPLE 2

A preferred cream formulation was prepared in the following manner: (1) Cetyl or myristyl alcohol (4%), glyceryl stearate (acid stable) (2.6%), stearyl octanoate (2.4%), octyl methoxycinnamate (3%), caprylic/capric triglycerides (2.6%), dimethicone (2.6%), lanolin anhydrous (3.2%) and stearic acid or cetyl stearyl alcohol/PEG 20 stearate (2.6%) were combined and melted at a temperature not exceeding 70°C until homogeneous. (2) When molten, cetyl phosphate (2.6%) was added, then half of the deionized water (16.0%) (at approximately 70°C) was added while mixing, resulting in formation of an emulsion. (3) Allantoin (0.2%) and the remaining deionized water (16.0%) (cold) was then added while mixing. (4) The emulsion was cooled to about cooted to about 50°C, followed by addition of sorbitol (3%), laneth-40 (3%), chitin liquid (polysaccharide glucosamine, for enhanced spreadability) (1%), urea (10%), benzophenone-4 (2.4%), lactic acid (2.4%), triethanolamine (2.4%), 70% micronized zinc oxide in caprylic/capric triglycerides or octyl dodecyl neopentanoate (11%), 70% micronized titanium dioxide in caprylic/capric triglycerides or octyl dodecyl neopentanoate (5%), 50% micronized iron oxide (red) in propylene glycol (0.2%), and triethanolamine (2.6%) to form an emulsion. (5) The emulsion was cooled to 40°C, followed by addition of phenonip (0.4%), octhilinone (0.1%), *Aloe barbedensis* clear extract (2%), *Mimosa tenuiflora* extract (1%), *Centella (Hydrocotyl) asiatica* extract (1%), oil of *Melaleuca ahernifolia* (2%) and banana (*Musa sapientum*) extract (0.4%). If necessary, the emulsion is homogenized.

### EXAMPLE 3

A preferred lotion formulation contains:

| Zinc oxide lotion formulation | | |
|---|---|---|
| COMPOUND | WEIGHT % | TYPE OF COMPOUND |
| Deionized water | 18.75 | Solvent |
| Zinc oxide | 12.5 | UV protector |
| Octyldodecyl neopentanoate | 0.5 | Emollient, skin conditioning agent |
| Urea | 12.5 | Skin conditioning agent |
| Sorbitol | 3.0 | Moisturizer |
| Octylmethoxy cinnamate | 2.5 | UV absorber |
| Stearyl octanoate | 3.0 | Emollient |
| Polyacrylamide/laureth-7 | 3.0 | Binder |
| Benzophenone-4 | 3.0 | UV absorber |
| Stearic acid | 2.4 | Emulsifying agent |
| Cetyl phosphate | 2.0 | Emulsifying agent |
| Laneth-40 | 1.5 | Surfactant |
| Aloe barbedensis extract | 2.0 | Biological additive |
| Mimosa tenuiflora extract | 1.0 | Biological additive |
| Oil of Melaleuca alternifolia (tea tree oil) | 2.0 | Biological additive |
| Dimethicone | 3.0 | Skin conditioning agent |
| Cetyl alcohol | 2.0 | Emulsifying agent |
| Triethanolamine | 1.25 | pH adjuster |
| Allantoin | 0.25 | Skin conditioning agent |
| Sodium benzoate | nominal | Preservative |
| Chloracetamide | nominal | |
| Natural rose | nominal | fragrance |

### EXAMPLE 4

A preferred lotion formulation was prepared in the following manner: (1) Cetyl or myristyl alcohol (4%), glyceryl stearate (acid stable) (2.4%), stearyl octanoate (3.0%) and octyl methoxycinnamate (2.5%) were combined and melted at a temperature not exceeding 70°C until homogeneous. (2) When molten, cetyl phosphate (2.0%) was added, then half of the deionized water (9.0%) (at approximately 70°C), allantoin (0.25%) and the remaining deionized water (9.75%) (cold) were added while mixing. (3) The mixture was cooled to about 50°C, and the following were added: sorbitol (3.0%), laneth-40 (1.5%), chitin liquid (polysaccharide glucosamine, for enhanced sproadability) (1.0%), urea (12.5%), benzophenone-4 (3.0%), octhilinone (0.1%), phenonip or octyldodecyl neopentanoate (0.5%), triethanolamine (1.25%), 70% micronized zinc oxide in caprylic/capric triglycerides (12.5%), 70% micronized titanium dioxide in caprylic/capric triglycerides (5.0%) and 50% micronized iron oxide (yellow) in propylene glycol (0.25%) to form an emulsion. (4) The emulsion was cooled to 40°C, followed by addition of *Aloe barbedensis* clear extract (2.0%), *Mimosa tenuiflora* extract (1.0%), *Centella (Hydrocotyl) asiatica* extract (1.0%) oil of *Melaleuca alternifolia* (2.0%), banana (*Musa sapientum*) extract (0.5%) and polyacrylamide/C13-14 isoparaffine/laureth-7 (3.0%). If necessary, the emulsion is homogenized.

### EXAMPLES 5 - 10

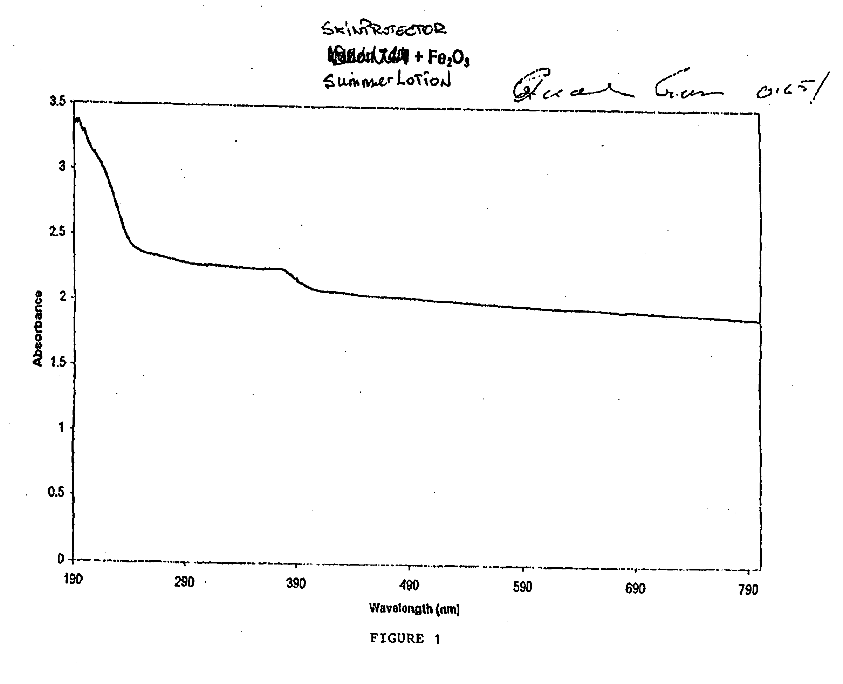

The data presented in Figures 1 through 5 indicate that the micronized zinc oxide compositions described herein function as skin protectors and block the transmittance of light in the UV, visible, and IR ranges.
Figure 1 illustrates a skin protector designated "Summer Lotion" plus iron (III) oxide and illustrates a plot of absorbance as a function of wavelength. (Ex. 5)

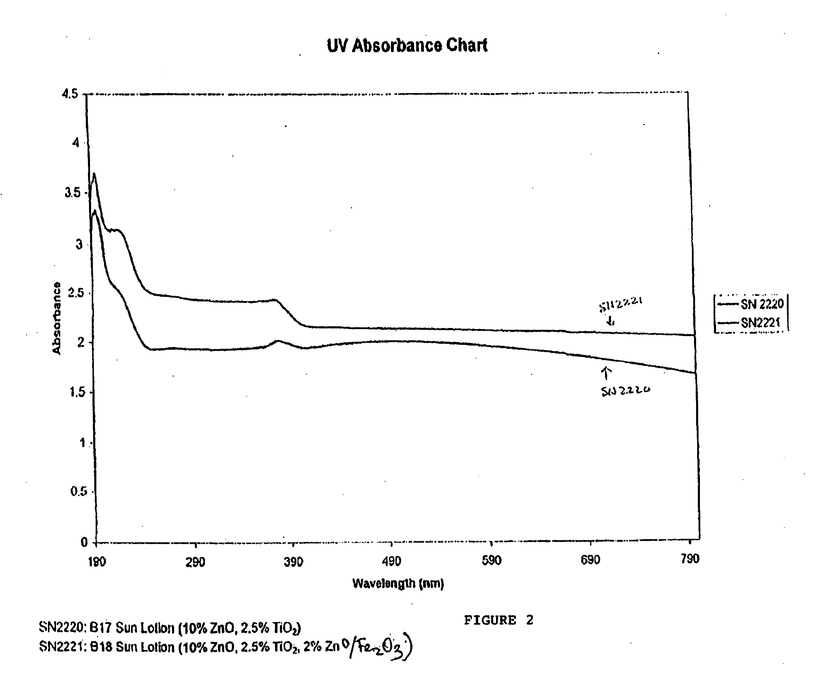

Figure 2 illustrates the ability of two micronized zinc oxide formulations B17 lotion (comprising 10% ZnO, 2.5% TiO₂) (SN 2200) (Ex. 6)and B18 lotion (comprising 10% ZnO, 2.5% TiO₂, and 2% ZnO/Fe₂O₃) (SN2221) (Ex. 7) to block and absorb wavelengths in the IR, visible, and UV ranges.

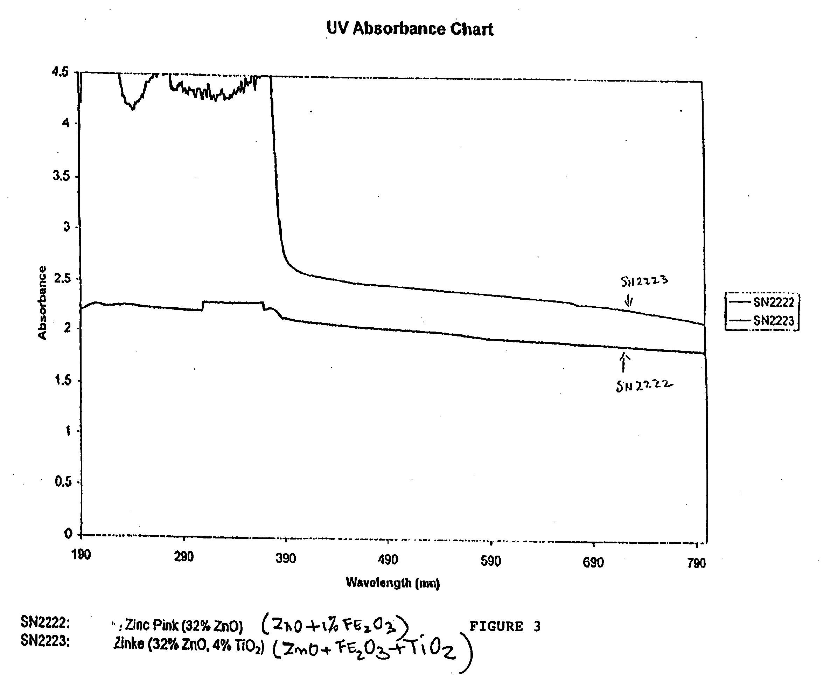

Figure 3 illustrates the ability of two micronized zinc oxide formulations designated "Zinc pink"-brand (comprising 32% ZnO, 1% Fe₂O₃) (SN 2222) (Ex. 8) and "Zince"-brand (comprising 32% ZnO, 1% Fe₂O₃, 4% TiO₂) (SN2223) (Ex. 9) to block and absorb wavelengths in the IR, visible, and UV ranges.

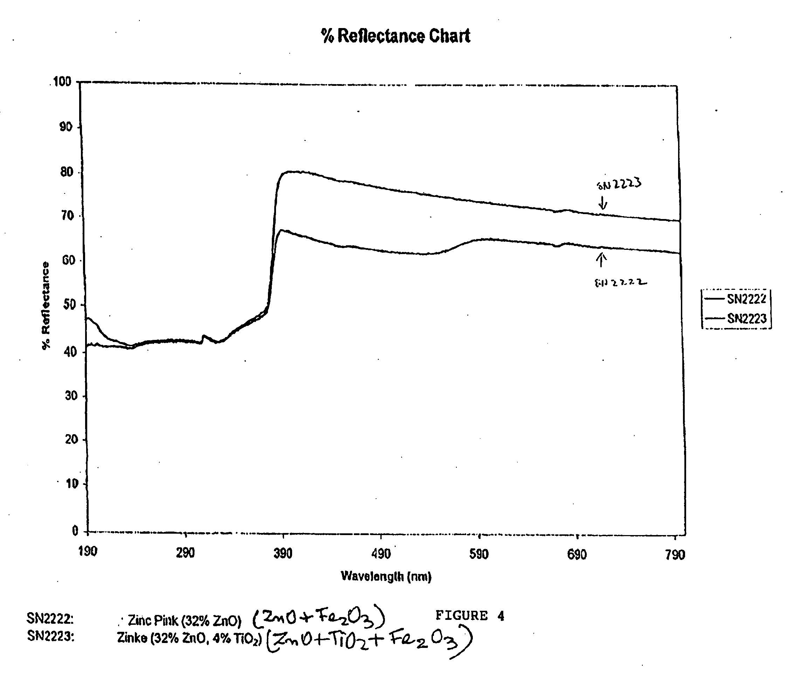

Figure 4 illustrates the ability of two micronized zinc oxide formulations designated "Zinc pink"-brand (comprising 32% ZnO, 1% Fe₂O₃) (SN 2222) (Ex. 8) and "Zince"-brand (comprising 32% ZnO, 1% Fe₂O₃, 4% TiO₂) (SN2223) (Ex. 9) to block and absorb wavelengths in the IR, visible, and UV ranges.

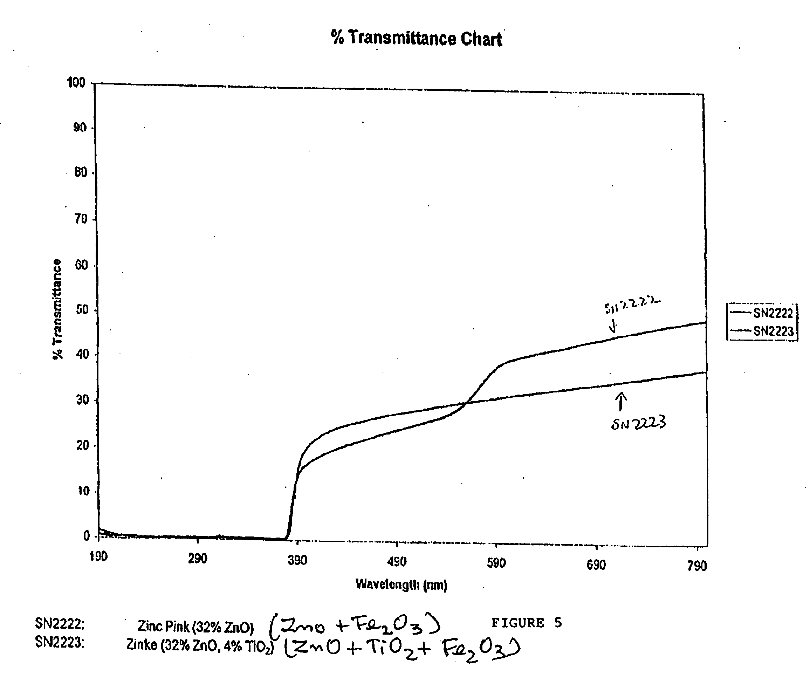

Figure 5 illustrates the ability of two micronized zinc oxide formulations designated "Zinc pink"-brand (comprising 32% ZnO, 1% Fe₂O₃) (SN 2222) (Ex. 8) and "Zince"-brand (comprising 32% ZnO, 1% Fe₂O₃, 4% TiO₂) (SN2223) (Ex. 9).

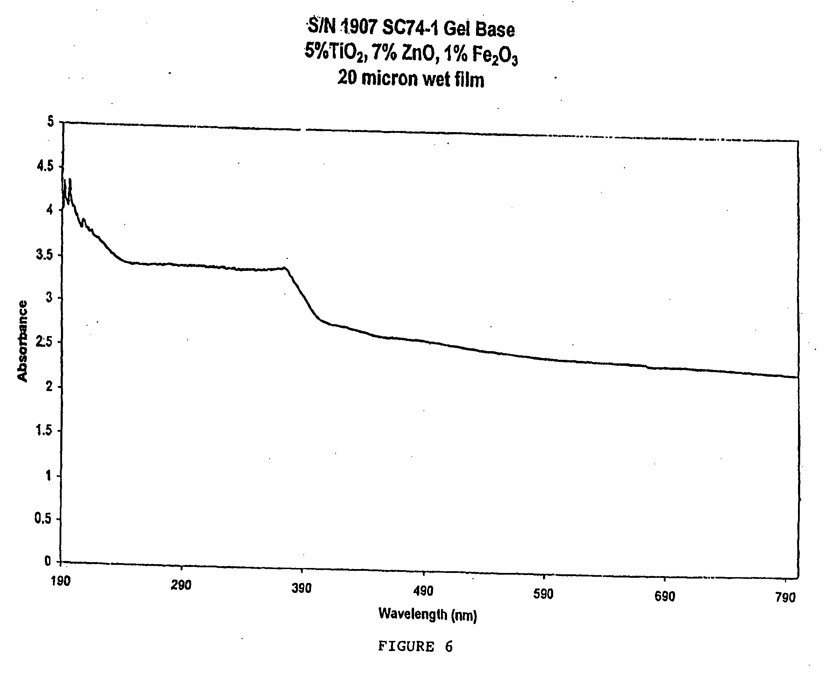

Figure 6 illustrates the ability of a micronized zinc oxide formulation designated SC74-1 Gel Base (comprising 7% ZnO, 5% TiO₂, 1% Fe₂O₃) (20 micron wet film) (Ex. 10) to block and absorb wavelengths in the IR, visible, and UV ranges.

Although the foregoing invention has been described in some detail by way of illustration and example for purposes of clarity of understanding, it is readily apparent to those of ordinary skill in the art in light of the teachings of this invention that certain changes and modifications, particularly with regard to specific exemplary components and to the specific ranges of the components of the formulations, may be made thereto without departing from the spirit and scope of that which is described and claimed.

## Claims

1. A topical sunscreen composition comprising between about 8% and 40% by weight micronized zinc oxide.

2. The composition of Claim 1, further comprising a hydrotrope and water.

3. The composition of Claim 1 or 2, wherein the zinc oxide is micronized by a "wet" micronization process.

4. The composition of Claims 1 or 2, wherein the mean particle size of the zinc oxide is less than approximately 5 µm.

5. The composition of Claims 1 or 2, wherein the mean particle size of the zinc oxide is between approximately 0.01 µm and approximately 1 µm.

6. The composition of Claim 2, further comprising the biological additive oil of Melaleuca alternifolia (Tea Tree Oil).

7. The composition of Claims 1 or 2, further comprising iron (III) oxide.

8. The composition of Claim 7, further comprising titanium oxide.

9. The composition of Claims 1 or 2, further comprising titanium oxide.

10. The composition of Claim 1, wherein said composition comprises between about 10% and 20% by weight micronized zinc oxide.

11. The composition of Claim 1, wherein said composition comprises about 10% by weight micronized zinc oxide.

12. The composition of Claim 1, wherein said composition comprises about 32% by weight micronized zinc oxide.

13. The composition of Claim 10, wherein said composition comprises between about 10% and 15% by weight micronized zinc oxide.

14. The composition of Claim 10, wherein said composition comprises between about 12.5% by weight micronized zinc oxide.

15. The composition of Claim 1, wherein said zinc oxide is micronized in oil.

16. The composition of Claim 15, wherein said oil is derived from plant materials.

17. The composition of Claim 16, wherein said oil comprises capric/caprylic triglycerides.

18. The composition of Claim 17, wherein said zinc oxide is prepared by a wet micronization process.

19. A method of protecting livestock from UV irradiation, comprising the step of topically applying the composition of Claim 1 to said livestock.

20. The method of Claim 19, wherein said livestock are mammals.

21. A method of preparing a medicament for the treatment of an individual having prematurely aged, photo-damaged or heat damaged skin, comprising combining the composition of Claims 1, 2, 6, or 10-15 with a suitable carrier or diluent.

22. The method of Claim 21, wherein the zinc oxide of the composition is prepared by a wet micronization process.
